(19) Europäisches Patentamt
European Patent Office
Office européen des brevets

(11) Veröffentlichungsnummer: **0 073 393**
**A1**

# (12) EUROPÄISCHE PATENTANMELDUNG

(21) Anmeldenummer: **82107419.2**

(22) Anmeldetag: **16.08.82**

(51) Int. Cl.³: **C 07 D 277/18**, A 61 K 31/425

(30) Priorität: **27.08.81 DE 3133918**

(43) Veröffentlichungstag der Anmeldung: **09.03.83**
**Patentblatt 83/10**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Behner, Otto, Dr., In den Birken 83, D-5600 Wuppertal 1 (DE)**
Erfinder: **Stendel, Wilhelm, Dr., In den Birken 55, D-5600 Wuppertal 1 (DE)**
Erfinder: **Andrews, Peter, Dr., Gellertweg 2, D-5600 Wuppertal 1 (DE)**

(54) **2-Arylhydrazino-2-thiazoline, Acylderivate derselben, 2-Arylazo-2-thiazoline, Herstellungsverfahren und ihre Verwendung zur Bekämpfung von Ekto- und Endoparasiten.**

(57) Thiazolinderivate der Formel

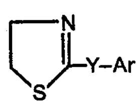

in der Y für –N=N– oder 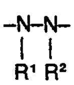 steht und R¹ und R² Wasserstoff oder Acyl bedeuten mit der Maßgabe, daß mindestens einer der Substituenten R¹ oder R² für Wasserstoff steht, und Ar einen disubstituierten oder mit einem weiteren Ring anellierten Phenylrest bedeutet, mit der Maßgabe, daß die Substituenten nicht in 2,6-Stellung stehen, bzw. deren Salze; Verfahren zu ihrer Herstellung und ihre Verwendung zur Bekämpfung von Ektoparasiten und Endoparasiten.

EP 0 073 393 A1

0073393

BAYER AKTIENGESELLSCHAFT 5090 Leverkusen, Bayerwerk

Zentralbereich
Patente, Marken und Lizenzen Ad/Kü-c

# 2-Arylhydrazino-2-thiazoline, Acylderivate derselben, 2-Arylazo-2-thiazoline, Herstellungsverfahren und ihre Verwendung zur Bekämpfung von Ekto- und Endoparasiten

Die Erfindung betrifft neue 2-Arylhydrazino-2-thiazoline, Acylderivate derselben und neue 2-Arylazo-2-thiazoline, Verfahren zu ihrer Herstellung und ihre Verwendung zur Bekämpfung von Ektoparasiten und Endoparasiten.

Es ist bereits bekannt, daß im Benzolring monosubstituierte oder in 2,6-Stellung disubstituierte 2-Phenylhydrazino-2-thiazoline, 2-Phenylazo-2-thiazoline sowie gewisse Acylderivate eine anthelmintische, antihypertensive und ektoparasitäre Wirkung entfalten. (M.T. Wu et al., Journ. pharmac. Sci, 66, 1150 (1977); US-Patent 4 046 753, Anmelder Merck & Co, Inc).

Es wurde nun gefunden, daß die neuen Thiazoline der Formel I

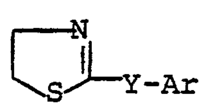 (I)

in der Y für -N=N- oder 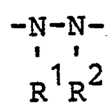

steht und $R^1$ und $R^2$ Wasserstoff oder Acyl bedeuten mit der Maßgabe, daß mindestens einer der Substituenten $R^1$ oder $R^2$ für Wasserstoff steht und Ar einen disubstituierten oder mit einem weiteren Ring anellierten Phenylrest bedeutet, mit der Maßgabe, daß die Substituenten nicht in 2,6-Stellung stehen, bzw. deren Salze hervorragend geeignet sind zur Bekämpfung von tierischen Ektoparasiten, insbesondere aus der Klasse der Akariden, sowie von Endoparasiten, insbesondere Helminthen.

Bei den Acylresten handelt es sich bevorzugt um gegebenenfalls substituierte $C_1$-$C_6$-Alkanoylreste, gegebenenfalls substituierte $C_1$-$C_6$-Alkenoylreste, gegebenenfalls substituierte $C_7$-$C_{10}$-Aroylreste, $C_2$-$C_6$-Alkoxycarbonyl- oder Carbamoylreste, $C_1$-$C_6$-Alkylsulfonylreste oder gegebenenfalls durch $C_1$-$C_2$-Alkylgruppen, $NO_2$-Gruppen oder Halogen ein- oder mehrfach substituierte Arylsulfonylreste.

Besonders bevorzugte Acylreste sind Formyl-, Acetyl-, Trichloracetyl-, Trifluoracetyl-, Propionyl-, Crotonyl-, Benzoyl-, Chlorbenzoyl-, Ethoxycarbonyl-, Methoxycarbonyl-, N-Methylcarbamoyl-, N-Phenylcarbamoyl-, Methansulfonyl-, Ethansulfonyl-, Benzolsulfonyl-, Chlorbenzolsulfonyl-, Toluolsulfonyl-, Nitrobenzolsulfonyl- oder Naphthalinsulfonylreste.

Bei dem Rest Ar handelt es sich um durch niederes Alkyl, Halogen, niederes Alkoxy oder Trifluormethyl

vorzugsweise in 2,3-Stellung disubstituiertes Phenyl, um Naphthyl, Tetrahydronaphthyl oder Indanyl.

Besonders bevorzugt ist der Rest Ar ein 2,3-Dimethylphenyl-, 3-Chlor-2-methylphenyl-, 2-Methoxy-3-methylphenyl-, 2-Methyl-3-trifluormethylphenyl-, 3-Chlor-2-isopropylphenyl-, 2,3-Dichlorphenyl-, 1-Naphthyl-, 5,6,7,8-Tetrahydro-1-naphthyl oder 4-Indanylrest.

Weiterhin wurde gefunden, daß man die erfindungsgemäßen Verbindungen der Formel I, in der Y für die Gruppe -NH-NH- steht, erhält, wenn man

a) Arylhydrazine der Formel II

$$Ar-NH-NH_2 \qquad (II)$$

in der Ar die oben angegebene Bedeutung hat, mit Thiazolinen der Formel III

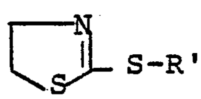

(III)

in der R' einen gegebenenfalls substituierten Alkylrest bedeutet, in Gegenwart von starken Säuren umsetzt, oder

b) Thiosemicarbazide der Formel IV,

$$Ar-NH-NH-CS-NH-CH_2-CH_2-X \qquad (IV)$$

in der X eine Hydroxylgruppe, eine Alkylsulfonyloxy- oder Arylsulfonyloxygruppe oder ein Halogenatom bedeutet, gegebenenfalls in Gegenwart einer starken Säure cyclisiert, oder

c) Isothiosemicarbazide der Formel V gegebenenfalls in Gegenwart einer starken Säure cyclisiert.

$$\begin{array}{l} Ar\text{-}NH\text{-}NH\text{-}C{=}NH \\ \qquad\qquad\quad\ \ | \\ \qquad\qquad\quad\ S\text{-}CH_2\text{-}CH_2\text{-}NH_2 \end{array} \qquad (V)$$

Die Thiosemicarbazide der Formel IV erhält man auf an sich bekannte Weise durch Umsetzung von Isothiocyanaten der Formel VI

$$X\text{-}CH_2\text{-}CH_2\text{-}NCS \qquad (VI)$$

mit Arylhydrazinen der Formel II.

Die Isothiosemicarbazide der Formel V erhält man auf an sich bekannte Weise durch Umsetzung eines aus Arylhydrazinen der Formel II mit Rhodanwasserstoffsäure erhaltenen Thiosemicarbazide der Formel VII

$$Ar\text{-}NH\text{-}NH\text{-}CS\text{-}NH_2 \qquad (VII),$$

in der Aryl die oben angegebene Bedeutung hat, mit z.B. 2-Chlorethylamin oder 2-Bromethylamin.

Die Acylierung der Verbindungen der Formel I, in denen $R^1$ und $R^2$ Wasserstoff bedeutet, zu Verbindungen der Formel I, in denen $R^1$ oder $R^2$ ein Acylrest der oben angegebenen Bedeutung ist, wird auf an sich bekannte Weise durchgeführt, beispielsweise indem man die nicht acylierten Verbindungen in einen geeigneten Lösungsmittel oder Lösungsmittelgemisch in Gegenwart eines Säurefängers mit Sulfonsäurehalogeniden, Carbonsäurehalogeniden oder -anhydriden oder Chlorameisensäurehalogeniden oder -anhydriden zur Reaktion bringt oder mit Isocyanaten in Abwesenheit einer Base reagieren läßt.

Als Säurefänger eingesetzte Basen können sein z.B. Alkali- oder Erdalkali-hydroxide, -carbonate oder -hydrogencarbonate oder tertiäre Amine wie Triethylamin oder Pyridin.

Weiterhin wurde gefunden, daß man die erfindungsgemäßen Verbindungen der allgemeinen Formel I, in der Y für eine Azogruppe steht und Ar die oben angegebene Bedeutung hat, erhält, wenn man

a) Verbindungen der Formel I, in der Y für eine Hydrazinogruppe steht und Ar die oben angegebene Bedeutung hat, dehydriert mit Hilfe von Oxidationsmitteln wie Mennige, Silberoxid, Chromsäure, Wasserstoffperoxid oder auch Luftsauerstoff, oder

b) Verbindungen der Formel I, in der Y für eine sulfonylierte Hydrazinogruppe steht und Ar die oben angegebene Bedeutung hat, der Abspaltung

des Sulfonylrests als Sulfinsäure unterwirft. Diese Abspaltung erfolgt z.B. schon unter den Bedingungen der Synthese dieser Verbindungen; es kann jedoch vorteilhaft sein, die Reaktion bei erhöhter Temperatur durchzuführen.

Beispiele für bevorzugte erfindungsgemäße Verbindungen sind:

2-[2-(2,3-Dimethylphenyl)hydrazino]-2-thiazolin
2-[2-(3-Chlor-2-methylphenyl)hydrazino]-2-thiazolin
2-[2-(2-Methoxy-3-methylphenyl)hydrazino]-2-thiazolin
2-[2-(2-Methyl-3-trifluormethylphenyl)hydrazino]-2-thiazolin
2-[2-(3-Chlor-2-isopropylphenyl)hydrazino]-2-thiazolin
2-[2-(2,3-Dichlorphenyl)hydrazino]-2-thiazolin
2-[2-(1-Naphthyl)hydrazino]-2-thiazolin
2-[2-(5,6,7,8-Tetrahydro-1-naphthyl)hydrazino]-2-thiazolin
2-[2-(2,3-Dimethylphenyl)-1-(oder 2)-phenylsulfonylhydrazino]-2-thiazolin
2-[2-(1-Naphthyl)-1-(oder 2)-phenylsulfonylhydrazino]-2-thiazolin
2-[2-(5,6,7,8-Tetrahydro-1-napthyl)-1-(oder 2)-phenylsulfonylhydrazino]-2-thiazolin
2-[2-(2,3-Dimethylphenyl)-1-(oder 2)-methylsulfonylhydrazino]-2-thiazolin
2-[2-(4-Indanyl)-hydrazino]-2-thiazolin
2-[2-(2,3-Dimethylphenyl)-1-(oder 2)-(4-chlorphenylsulfonyl)hydrazino]-2-thiazolin

2-/2-(2,3-Dimethylphenyl)-1-(oder 2)-(4-nitrophenyl-sulfonyl)hydrazino7-2-thiazolin
2-/2-(2,3-Dimethylphenyl)-1-(oder 2)-(4-tolylsulfonyl)hydrazino7-2-thiazolin
2-(2,3-Dimethylphenylazo)-2-thiazolin
2-/5,6,7,8-Tetrahydro-1-napthylazo)-2-thiazolin
2-(1-Naphthylazo)-2-thiazolin
2-(3-Chlor-2-methylphenylazo)-2-thiazolin
2-(2,3-Dichlorphenylazo)-2-thiazolin.

Die neuen Wirkstoffe sowie gegebenenfalls ihre Salze weisen starke acarizide Wirkung auf, besonders gegen Acariden, die als tierische Ektoparasiten domestizierte Tiere, wie Rinder, Schafe und Kaninchen befallen. Gleichzeitig haben die erfindungsgemäßen Verbindungen eine geringe Warmblütertoxizität. Sie eignen sich daher gut zur Bekämpfung von tierischen Ektoparasiten aus der Klasse der Acariden. Darüber hinaus haben sie jedoch auch Wirkung gegen andere Acariden sowie gegen Insekten.

Als wirtschaftlich wichtige Ektoparasiten, die besonders in tropischen und subtropischen Ländern eine große Rolle spielen, seien genannt: die australische und südamerikanische Rinderzecke Boophilus microplus, die südafrikanische Rinderzecke Boophilus decoloratus, beide aus der Familie der Ixodidae, sowie Rinder- und Schafzecken der Genera Rhipicephalus, Amblyomma, Hyalomma.

Im Laufe der Zeit sind insbesondere Zecken gegen die als Bekämpfungsmittel bisher verwendeten Phosphorsäureester

und Carbamate resistent geworden, so daß der Bekämpfungserfolgt in vielen Gebieten in wachsendem Maße infrage gestellt wird. Zur Sicherung einer wirtschaftlichen Viehhaltung in den Befallsgebieten besteht ein dringender Bedarf an Mitteln, mit denen alle Entwicklungsstadien, also Larven, Nymphen, Metanymphen und Adulti auch resistenter Stämme, beispielsweise des Genus Boophilus sicher bekämpft werden können. In hohem Maße gegen die bisher verwendeten Phosphorsäureester resistent sind beispielsweise in Australien der Mackay-Stamm, der Biarra-Stamm und der Mt-Alford-Stamm von Boophilus microplus.

Die erfindungsgemäßen Wirkstoffe sind sowohl gegen die normalempfindlichen, als auch gegen die resistenten Stämme, z.B. von Boophilus, gleich gut wirksam. Sie wirken in üblicher Applikation am Wirtstier sowohl direkt auf alle am Tier parasitierenden Formen als auch stark ovizid auf die adulten Formen, so daß der Vermehrungscyclus der Zecken sowohl in der parasitischen Phase auf dem Tier als auch in der nicht parasitären Phase unterbrochen wird. Die Eiablage wird unterbunden, die Entwicklung und das Schlüpfen inhibiert. Hervorzuheben sind insbesondere der schnell eintretende erregende Effekt auf alle parasitierenden Formen, die ihre Saughaltung aufgeben, in unphysiologischer Weise auf dem Wirtstier umherwandern, abfallen und schließlich sterben (detaching effect) sowie insbesondere auch die gute Wirkung gegen die erfahrungsgemäß schwer bekämpfbaren Metanymphenstadien.

Ferner wirken sie in der gleichen Art auf alle Entwicklungsstadien mehrwertiger Zecken wie z.B. Amblyomma spp., Hyalomma spp., Rhipicephalus spp., Ixodes spp., Hämaphysalis spp., Dermacentor spp.

Ein detaching effect zeigt sich auch bei Insekten, beispielsweise bei Läusen, wie Hämatopinus spp.

Die neuen Verbindungen der Formel I sind darüber hinaus auch gegen Endoparasiten wirksam. Sie wirken vor allem gegen Magen- und Darmnematoden der Wiederkäuer und Fleischfresser, auch solche, die gegen die gebräuchlichen Benzimidazolanthelmintika resistent und damit nicht mehr ausreichend therapierbar sind.

Sie wirken vorzugsweise gegen Haemonchus in besonders niedriger Dosierung.

Die Wirkung wurde im Tierversuch nach oraler, subcutaner und dermaler Applikation bei stark mit Parasiten befallenen Versuchstieren geprüft. Die angewendeten Dosierungen wurden sehr gut von den Versuchstieren vertragen.

Die Verbindungen wirken nicht nur nach oraler oder parenteraler Verabreichung, sondern auch gut nach dermaler Applikation (Pour-On, Spot-On). Damit werden Verabreichungsweisen möglich, die bei den im Handel befindlichen nur nach oraler Gabe wirkenden Benzimidazol-Anthelmintika nicht möglich sind, andererseits aber für den Anwender vorteilhaft sind wie z.B. die dermale oder subcutane Behandlung.

Die neuen Verbindungen können als Anthelmintika sowohl in der Human- als auch in der Veterinärmedizin verwendet werden. Sie können in bekannter Weise in die üblichen Formulierungen übergeführt werden.

Je nach der vorgesehenen Applikationsform können die neuen Wirkstoffe in die praxisüblichen Formulierungen übergeführt werden, wie beispielsweise Lösungen, Emulsionen, Suspensionen, Pulver, Pasten und Granulate.

Diese werden in bekannter Weise hergestellt, z.B. durch Vermischen der Wirkstoffe mit Streckmitteln, d.h. flüssigen Lösungsmitteln und/oder Trägerstoffen, gegebenenfalls unter Mitverwendung von oberflächenaktiven Mitteln, also Emulgier- und/oder Dispergiermitteln, wobei z.B. im Falle der Verwendung von Wasser als Streckmittel gegebenenfalls organische Lösungsmittel als Hilfslösungsmittel verwendet werden können.

Als Lösungsmittel kommen z.B. infrage: Aromaten (z.B. Xylol, Benzol, Orthodichlorbenzol, Trichlorbenzol), Paraffine (z.B. Erdölfraktionen), Alkohole (z.B. Methanol, Ethanol, Isopropanol, Butanol), stark polare Lösungsmittel wie Dimethylformamid, N-Methyl-pyrrolidon, Dimethylsulfoxid sowie auch Wasser.

Als feste Trägerstoffe seien genannt: natürliche Gesteinsmehle (z.B. Kaoline, Tonerden, Talkum, Kreide) synthetische anorganische Trägerstoffe (z.B. hochdisperse Kieselsäure, Silikate); als Emulgiermittel: sowohl nichtionogene als auch anionische oder kationische Emulgatoren wie z.B. Polyoxyethylen-Fettsäureester, Polyoxyethylen-Fettalkoholether, z.B. Alkyl-aryl-polyglykolether; Alkylsulfonate und Arylsulfonate, quartäre Ammoniumsalze mit längeren Alkylresten. Als Dispergiermittel seien genannt: Lignin, Sulfitablaugen und Methylcellulose.

Die Formulierungen enthalten im allgemeinen zwischen 0,1 und 95 Gew.-% Wirkstoff, vorzugsweise zwischen 0,5 bis 90 Gew.-%. Die Anwendungskonzentrationen werden aus den Formulierungen (s.o.) durch Verdünnen mit Wasser hergestellt. Sie können, je nach der Anwendungsform, in einem großen Bereich variiert werden und liegen zwischen 1 und 50 000 ppm (g/g), vorzugsweise zwischen 5 und 500 ppm.

Die Applikation erfolgt in üblicher Weise, z.B. durch Besprühen (spray), Gießen (pour on), Vernebeln oder durch Bad (dip).

Den Formulierungen oder den anwendungsfertigen Lösungen können noch sonstige Hilfsmittel oder Wirkstoffe, wie Desinfektionsmittel oder speziell geeignete Insektizide, zugemischt werden.

Die wäßrigen Lösungen bzw. Emulsionen der erfindungsgemäßen Wirkstoffe besitzen unter Praxisbedingungen eine gute Stabilität, so daß die gebrauchsfertigen Anwendungsformen auch bei längerem Stehen und in einem pH-Bereich von 7 - 9 drei Monate und länger wirksam bleiben.

Beispiel 1

2-[2-(2,3-Dimethylphenyl)-hydrazino]-2-thiazolin

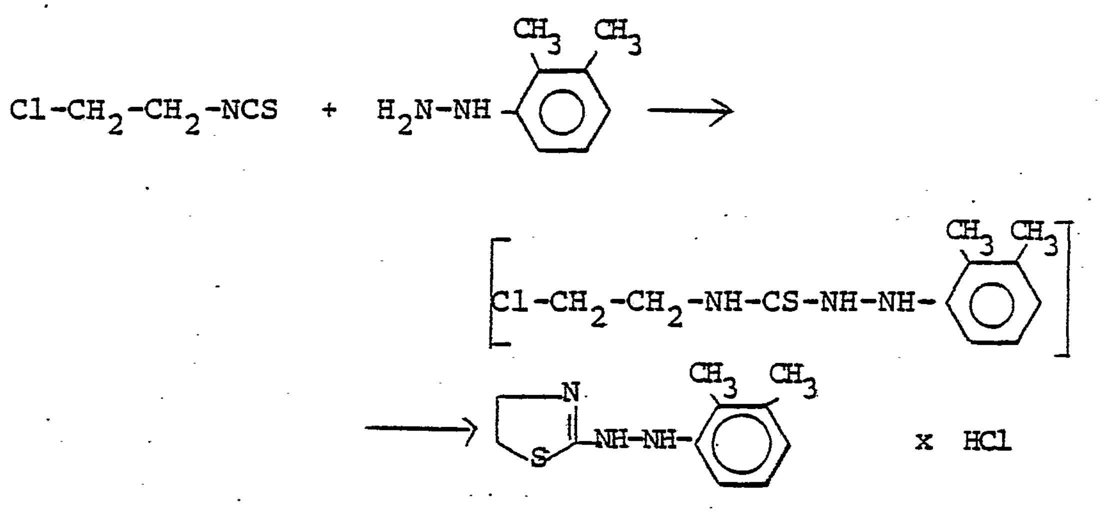

Man löst 13,6 g (0,1 Mol) 2,3-Dimethylphenylhydrazin in 50 ml Toluol und versetzt unter Rühren und Kühlung mit Eiswasser innerhalb von 15 Minuten mit einer Lösung von 12,8 g (5 % Überschuß) 2-Chlorethylisothiocyanat in 25 ml Toluol, rührt 2 Stunden bei Raumtemperatur und 2 Stunden unter R.F. nach. Man saugt kalt ab und kristallisiert aus Isopropanol um. Fp. 220 - 221° (Z.) (Hydrochlorid), 13,5 g (52,5 % der Theorie).

Dieselbe Verbindung erhält man, wenn man 2,3-Dimethylphenylhydrazin und 2-Methylthio-2-thiazolin-hydrochlorid in Xylol 2 Stunden auf 120 - 130° erhitzt.

Beispiel 2

2-[2-(3-Chlor-2-tolyl)hydrazino]-2-thiazolin

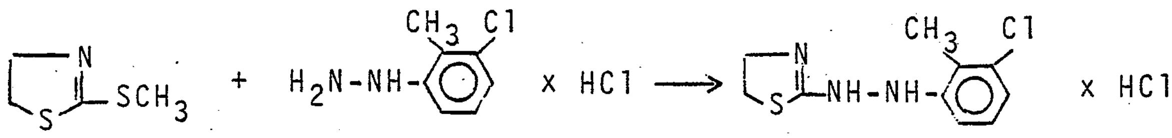

Eine unter Rückfluß gerührte Suspension aus 15,5 g (o,o8 Mol) 3-Chlor-2-tolylhydrazin-hydrochlorid in 1oo ml Isopropanol versetzt man langsam binnen 3o Minuten mit einer Lösung von 11,2 g (5 % Überschuß) 2-Methylthio-2-thiazolin in 2o ml Isopropanol.

Gegen Ende der Zugabe liegt klare Lösung vor, aus der bald neue Kristallisation erfolgt. Man rührt 3 Stunden unter Rückfluß nach, saugt nach dem Abkühlen ab, wäscht mit Isopropanol und Ether nach und trocknet. Fp. 24o - 245$^{o}$ (Z.) (Hydrochlorid), 21,5 g (96,5 % d. Theorie).

Beispiel 3

2-/2-(1-Naphthyl)hydrazino/-2-thiazolin

Analog Beispiel 1 aus 1-Naphthylhydrazin und 2-Chlorethylisothiocyanat. Fp. 222° (Z.) (Hydrochlorid).

Beispiel 4

2-/2-(5,6,7,8-Tetrahydro-1-naphthyl)hydrazino/-2-thiazolin

Analog Beispiel 1 aus 5,6,7,8-Tetrahydro-1-naphthylhydrazin und 2 -Chlorethylisothiocyanat. Fp. 238 - 240° (Z.) (Hydrochlorid).

Beispiel 5

2-/2-(2,3-Dimethylphenyl)-1-(oder 2)-phenylsulfonylhydrazino/-2-thiazolin

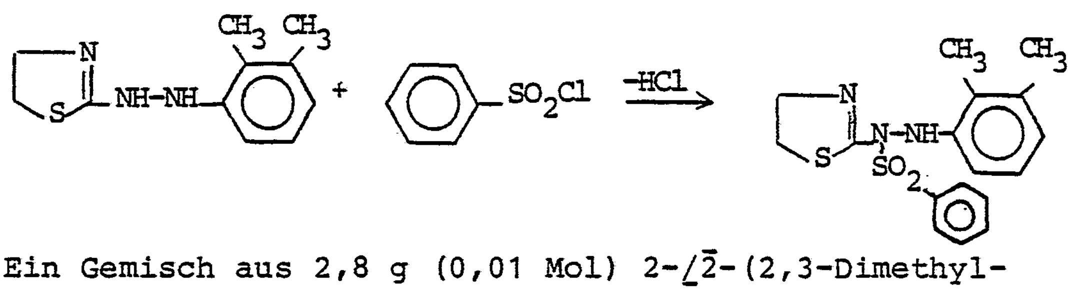


Ein Gemisch aus 2,8 g (0,01 Mol) 2-/2-(2,3-Dimethylphenyl)-hydrazino/-2-thiazolin-hydrochlorid in 20 ml Methylenchlorid und 1,2 g (0,03 Mol) Natriumhydroxid in 5 ml Wasser wird unter gutem Rühren innerhalb von 15 Minuten versetzt mit einer Lösung von 1,94 g (10 % Überschuß) Benzolsulfonylchlorid in 10 ml Methylen-chlorid. Man rührt 1 Stunde bei Raumtemperatur nach, verdünnt mit etwas Wasser, trennt die Phasen, trocknet die organische Phase über Kaliumcarbonat und dampft zur Trockne ein. Das rote Rohprodukt wird mit 15 ml Ethanol kurz aufgekocht. Nach dem Abkühlen und Absaugen farblose Kristalle, 2,9 g (80 % der Theorie), Fp. 121 - 124° (Z.).

Beispiel 6

2-[2-(1-Naphthyl)-1-(oder 2)-phenylsulfonylhydrazino]-2-thiazolin

Analog Beispiel 4 aus 2-[2-(1-Naphthyl)hydrazino]-2-thiazolin und Benzolsulfonylchlorid.

Beispiel 7

2-(2,3-Dimethylphenylazo)-2-thiazolin

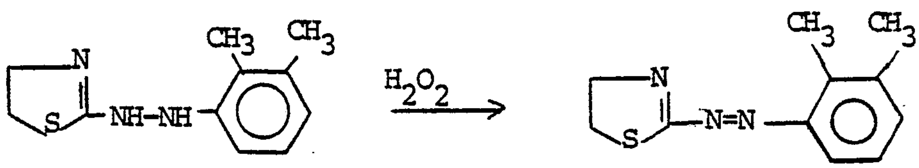


Ein Gemisch aus 6,5 g (0,025 Mol) 2-[2-(2,3-Dimethylphenyl)-hydrazino]-2-thiazolin-hydrochlorid, 60 ml Methylenchlorid und 50 ml 2n Natronlauge wird mit 3,5 ml 30 prozentigem Perhydrol versetzt. Man rührt 18 Stunden unter Rückfluß und gibt nach 6 Stunden 2 ml und nach 12 Stunden 1 ml Perhydrol nach. Man trennt die organische Phase ab, trocknet über Kaliumcarbonat,

dampft ein und kristallisiert den Rückstand aus Hexan-Diethylether um.
Fp. 74 - 79°, Ausbeute 4,1 g (74 % der Theorie).

Die Dehydrierung läßt sich auch mit gleichem Erfolg mit anderen Oxidationsmitteln, z.B. Chromsäure, durchführen.

## Beispiel 8

2-(3-Chlor-2-tolylazo)-2-thiazolin
Analog Beispiel 7 aus 2-[2-(3-Chlor-2-tolyl)hydrazino]-2-thiazolin, Fp. 96 - 1o2°.

## Beispiel 9

2-(5,6,7,8-Tetrahydro-1-naphthylazo)-2-thiazolin
Analog Beispiel 7 aus 2-[2-(5,6,7,8-Tetrahydro-1-naphthyl)hydrazino]-2-thiazolin.

Zeckentest

Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

In diese Wirkstoffzubereitungen werden adulte, vollgesogene Zeckenweibchen der Art Boophilus microplus (resistent) eine Minute lang getaucht. Nach dem Tauchen von je 10 weiblichen Exemplaren überführt man diese in Petrischalen, deren Boden mit einer entsprechend großen Filterscheibe belegt ist.

Nach 20 Tagen wird die Wirksamkeit der Wirkstoffzubereitung bestimmt durch Ermittlung der Hemmung der Eiablage gegenüber unbehandelten Kontrollzecken. Die Wirkung drückt man inProzent aus, wobei 100 % bedeutet, daß keine Eier mehr abgelegt wurden und 0 % besagt, daß die Zecken Eier in normaler Menge ablegten.

Untersuchter Wirkstoff, geprüfte Konzentrationen, getestete Parasiten und erhaltene Befunde gehen aus der folgenden Tabelle hervor.

In-vitro-Test auf eiablagehemmende Wirkung an Zecken
(Boophilus microphus, Biarra-Stamm)

| Wirkstoff | 100 % | 50 % |
| --- | --- | --- |
| | Hemmung bei einer Wirkstoffkonzentration in ppm a.i. von | |
| 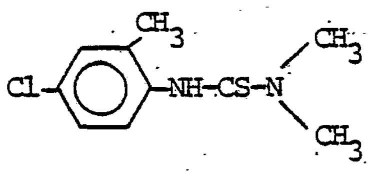 Chloromethiuron (bekanntes Vergleichspräparat) | - | >5000 |
| 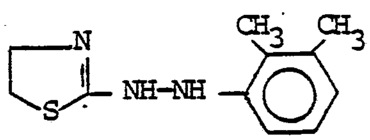 | 10 000 | 1000 |
| 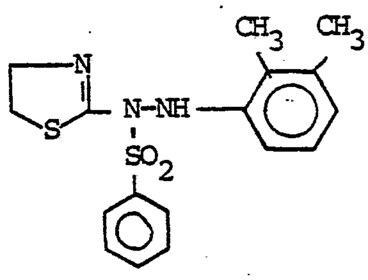 | >10 000 | 10 000 |
| 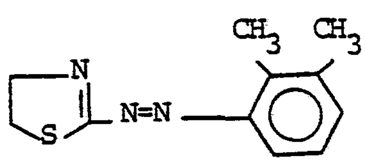 | 300 | 200 |

0073393

Magen- und Darmwurmtest/Schaf

Experimentell mit Haemonchus contortus infizierte Schafe wurden nach Ablauf der Präpatenzzeit der Parasiten behandelt. Die Wirkstoffmenge wurde als reiner Wirkstoff in Gelatinekapseln oral appliziert oder in einem geeigneten Lösungsmittel gelöst und subcutan oder dermal appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren können (Dosis effectiva). Geprüfte Wirkstoffe und wirksame Dosierungen (dosis effectiva minima) sind aus der nachfolgenden Tabelle ersichtlich.

| Wirkstoff | Applikations-weise | Dosis effectiva minima (Red ≤ 90 % in mg(kg) Haemonchus contortus |
|---|---|---|
| 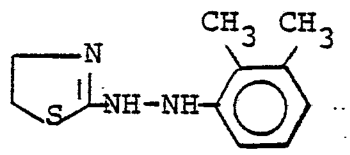 | per os | 1 |
| 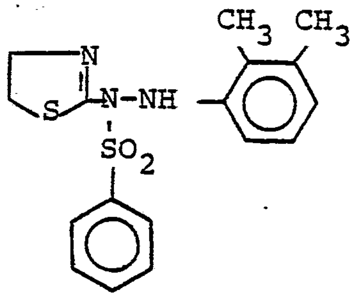 | per os | 1 |
| 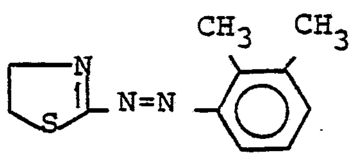 | per os | 0,5 |

## Patentansprüche

1. Thiazolinderivate der Formel

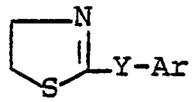

in der Y für -N=N- oder $-\underset{R^1}{N}-\underset{R^2}{N}-$ steht und $R^1$ und $R^2$ Wasserstoff oder Acyl bedeuten mit der Maßgabe, daß mindestens einer der Substituenten $R^1$ oder $R^2$ für Wasserstoff steht, und Ar einen disubstituierten oder mit einem weiteren Ring anellierten Phenylrest bedeutet, mit der Maßgabe, daß die Substituenten nicht in 2,6-Stellung stehen, bzw. deren Salze.

2. Thiazolinderivate nach Anspruch 1, dadurch gekennzeichnet, daß einer der Reste $R^1$ oder $R^2$ für einen gegebenenfalls substituierten $C_1$-$C_6$-Alkanoyl- gegebenenfalls substituierten $C_1$-$C_6$-Alkenoylrest, $C_7$-$C_{10}$-Aroyl-, $C_2$-$C_6$-Alkoxycarbonyl- oder Carbamoylrest, einen $C_1$-$C_6$-Alkylsulfonylrest oder einen gegebenenfalls durch $C_1$-$C_2$-Alkylgruppen oder Nitrogruppen oder Halogen ein- oder mehrfach substituierten $C_6$-$C_{10}$-Arylsulfonylrest steht und Ar durch $C_1$-$C_3$-Alkyl, Halogen, $C_1$-$C_3$-Alkoxy oder $-CF_3$ disubstituiertes oder mit einem weiteren Ring anelliertes Phenyl bedeutet.

3. Thiazolinderivate nach Anspruch 2, dadurch gekennzeichnet, daß der Rest Ar in 2- und 3-Stellung substituiert oder anelliert ist.

4. Verfahren zur Herstellung von Thiazolinderivaten der Formel

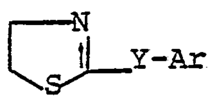 (I)

in der Y für 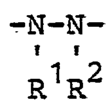

steht und $R^1$ und $R^2$ Wasserstoff bedeuten und Ar einen disubstituierten Phenylrest bedeutet oder mit einem weiteren Ring anelliertes Phenyl bedeutet, mit der Maßgabe, daß die Substituenten nicht in 2,6-Stellung stehen, dadurch gekennzeichnet, daß man

a) Arylhydrazine der Formel

$$Ar-NH-NH_2 \quad (II)$$

in der Ar die oben angegebene Bedeutung hat, mit Thiazolinen der Formel

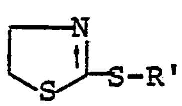 (III)

in der R' einen gegebenenfalls substituierten Alkylrest bedeutet, in Gegenwart von starken Säuren umsetzt, oder

b) Thiosemicarbazide der Formel

$$Ar-NH-NH-CS-NH-CH_2-CH_2-X \qquad (IV)$$

in der X eine Hydroxylgruppe, eine Alkylsulfonyloxy- oder Arylsulfonyloxygruppe oder ein Halogenatom bedeutet, gegebenenfalls in Gegenwart einer starken Säure cyclisiert, oder

c) Isothiosemicarbazide der Formel

$$\begin{array}{l} Ar-NH-NH-C=NH \\ \qquad\qquad\quad\; | \\ \qquad\qquad\quad\; S-CH_2-CH_2-NH_2 \end{array} \qquad (V)$$

gegebenenfalls in Gegenwart einer starken Säure cyclisiert.

5. Verfahren zur Herstellung von Thiazolinderivaten der Formel

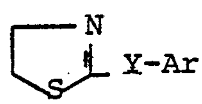

(I)

in der Y für $\begin{array}{c} -N-N- \\ \;\;|\;\;\;| \\ \;\;R^1R^2 \end{array}$

steht und einer der Reste $R^1$ und $R^2$ Wasserstoff, der andere Acyl bedeutet und Ar einen disubstituierten oder mit einem weiteren Ring anelliertes

Phenyl bedeutet, mit der Maßgabe, daß die Substituenten nicht in 2,6-Stellung stehen, dadurch gekennzeichnet, daß man Thiazolinderivate nach Anspruch 4 mit Säurehalogeniden, -anhydriden oder Isocyanaten zur Reaktion bringt.

6. Verfahren zur Herstellung von Thiazolinderivaten der Formel

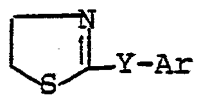

(I)

in der X für -N=N- steht und Ar einen disubstituierten oder mit einem weiteren Ring anellierten Phenylrest bedeutet, mit der Maßgabe, daß die Substituenten nicht in 2,6-Stellung stehen, dadurch gekennzeichnet, daß man Thiazolinderivate nach Anspruch 4, mit Hilfe eines Oxidationsmittels dehydriert, oder daß man Thiazolinderivate nach Anspruch 5, in denen der Acylrest ein Sulfonsäurerest ist, der Abspaltung des Sulfonylrests als Sulfinsäure unterwirft.

7. Mittel, gekennzeichnet durch einen Gehalt an mindestens einem Thiazolinderivat gemäß Anspruch 1.

8. Tickizides Mittel, gekennzeichnet durch einen Gehalt an mindestens einem Thiazolinderivat gemäß Anspruch 1.

9. Verfahren zur Herstellung von Mitteln, dadurch gekennzeichnet, daß man Thiazolinderivate gemäß

Anspruch 1 mit inerten, nichttoxischen, pharmazeutisch geeigneten Trägerstoffen vermischt.

10. Verwendung von Thiazolinderivaten gemäß Anspruch 1 bei der Herstellung von Mitteln gemäß Anspruch 7 und zur Bekämpfung von Endo- und/oder Ektoparasiten.

Europäisches Patentamt

**EUROPÄISCHER RECHERCHENBERICHT**

0073393

Nummer der Anmeldung

EP 82 10 7419.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 4 046 753 (M.H. FISHER et al.) | |
| D,Y | * Spalte 3, Zeilen 28 bis 46 ; Beispiele 1 bis 3 * | 1,4 |
| D,A | * Spalte 6, Zeile 65 bis Spalte 7, Zeile 16 * | 6-8 |
| | -- | |
| | DE - A1 - 2 457 309 (EGYT GYOGYSZERVEGYESZETI GYAR) | |
| Y | * Ansprüche 1, 4, 5 * | 1,4 |
| A | * Seite 3, letzter Absatz * | 3 |
| | -- | |
| A | DE - A1 - 2 511 731 (BAYER AG) * Ansprüche * | 1,7,8, 9 |
| | -- | |
| A | CH - A - 449 019 (CIBA AG) * Beispiel * | 1 |
| | ---- | |

KLASSIFIKATION DER ANMELDUNG (Int. Cl.3)

C 07 D 277/18
A 61 K 31/425

RECHERCHIERTE SACHGEBIETE (Int. Cl.3)

A 61 K 31/425
C 07 D 275/02
C 07 D 277/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 29-10-1982 | HASS |